# EUROPEAN PATENT APPLICATION

(11) **EP 4 177 349 A1**
(43) Date of publication of application: **10.05.2023**
(21) Application number: 22178717.9
(22) Date of filing: 13.06.2022
(51) Int. Cl.: C12P 13/04, C12P 13/00, C12P 7/40, C12P 7/62, C12N 1/18, C12R 1/865

(54) **SACCHAROMYCES CEREVISIAE AND FERMENTATION PRODUCT AND USE THEREOF**

(30) Priority: 05.11.2021 EP 21206607; 05.11.2021 CN 202111310132
(71) Applicant: Xiamen Oamic Biotechnology Co., Ltd., Xiamen, Fujian (CN)
(72) Inventor: Zeng, Shichao, Xiamen (CN); Zhao, Xiaolan, Xiamen (CN); Guo, Yashan, Xiamen (CN); Xing, Chenguang, Xiamen (CN); Liu, Gang, Xiamen (CN); Lin, Xiaohui, Xiamen (CN); Liu, Chaoxia, Xiamen (CN); Cai, Xiulian, Xiamen (CN)
(74) Representative: Zaboliene, Reda

(57) **Abstract**

The disclosure relates to a *Saccharomyces cerevisiae* strain OMK-77, deposited as CCTCC M 20211292 and a fermentation product and use thereof, and belongs to the technical field of fermentation engineering. The *Saccharomyces cerevisiae* or the fermentation product thereof could be used in a method for biodegrading L-tryptophan to prepare anthranilic acid, and make the method have the advantages such as fast reaction, highly safety, green and environmentally friendly process, fewer by-products, convenience in downstream extraction and separation, and high yield and high purity for the obtained anthranilic acid.

## Description

### Technical Field

The present disclosure relates to the technical field of fermentation engineering, and in particular to a *Saccharomyces cerevisiae* and a fermentation product and use thereof.

### Background

Methyl anthranilate has fruity, floral, powdery, musty and grape flavors, and naturally exists in grapes, strawberries, red wine and white wine, and is an important raw material for formulation of essences of types of grapes, citrus, loganberries, strawberries, watermelons and others. Methyl anthranilate is also a necessary raw material for preparing artificial orange oil. Thus, methyl anthranilate is in great demand in the market.

At present, methyl anthranilate in the market is mainly obtained by chemical synthesis. With the progress and development of the society, the will of people to return to nature is increasingly strong, which makes natural essences and fragrances more and more widely used in food industry. Compared with chemically synthesized products, the natural essences and fragrances made from natural substances through biotransformation have higher market acceptance and added value.

In the process of realizing the technical solutions of present disclosure, the inventor finds at least the following problems in the prior art:
The production of natural methyl anthranilate mainly depends on the oxidative demethylation reaction of methyl N-methyl anthranilate, which can be obtained from Egyptian petitgrain oil. However, the reaction depends on chemical oxidants such as MnO₂, which not only causes problems in the naturalness certification of the produced methyl anthranilate, but also produces a large number of wastes that are difficult to be processed, resulting in environmental pollution and producing a series of by-products which are disadvantageous to subsequent extraction and purification operations. Preparation of anthranilic acid via microbial fermentation is another possible way to prepare natural methyl anthranilate, but currently has no prospects for industrial application due to low space-time yield and low product concentration. For example, Victor E Balderas-Hernández et al. reported that the genetically modified *Escherichia coli* could be applied to produce anthranilic acid using glucose as a raw material, but only 14 g/L anthranilic acid could be produced by fermentation for 34 h. Jannis Kuepper et al. reported that only 1.54 g/L anthranilic acid could be obtained from glucose within 70 h by using genetically modified *Pseudomonas putida.*

Therefore, there is an urgent need for methods for preparing highly pure anthranilic acid and methyl anthranilate with a safe, green and environmentally friendly process, fewer by-products, convenience in downstream extraction and separation and high yield.

### Summary

A purpose of the present disclosure is to provide a *Saccharomyces cerevisiae* and a fermentation product and use thereof. In the first aspect, a *Saccharomyces cerevisiae* is provided. In the second aspect, a fermentation product of the *Saccharomyces cerevisiae* is provided. In the third aspect, a method for preparing the fermentation product is provided. In the fourth aspect, a method for preparing anthranilic acid is provided. In the fifth aspect, a method for preparing methyl anthranilate is provided. In the sixth aspect, use of the *Saccharomyces cerevisiae* of the first aspect or the fermentation product of the *Saccharomyces cerevisiae* of the second aspect is provided. The provided *Saccharomyces cerevisiae* has good catalytic activity and could be used to degrade L-tryptophan to prepare anthranilic acid. The obtained anthranilic acid has high purity, and the method has advantages of fast reaction, highly safety, green and environmentally friendly process, fewer by-products and convenience in downstream extraction and separation, fewer solvent residues, and high yield for anthranilic acid.

**The present disclosure** provides the following technical solutions.

In the first aspect, a *Saccharomyces cerevisiae* is provided.

A *Saccharomyces cerevisiae* having a collection number of CCTCC M 20211292 (collected at China Center for Type Culture Collection on October 18, 2021) is provided.

The *Saccharomyces cerevisiae* is named as *Saccharomyces cerevisiae* OMK-77_{∘}

In the second aspect, a fermentation product of the *Saccharomyces cerevisiae* is provided.

A fermentation product of the *Saccharomyces cerevisiae* of the first aspect is provided.

In some embodiments, the fermentation product may be a fermentation liquid, a fermentation supernatant, a cell suspension and/or an intracellular substance.

In the third aspect, a method for preparing the fermentation product is provided.

The method for preparing the fermentation product of the second aspect comprises: activating the *Saccharomyces cerevisiae* of the first aspect, subjecting the *Saccharomyces cerevisiae* to a at least one stage expanded culture, and then collecting fermentation product.

In some embodiments, the at least one stage expanded culture comprises a one-stage expanded culture, a two-stage expanded culture, a three-stage expanded culture or a four-stage expanded culture.

In the fourth aspect, a method for preparing anthranilic acid is provided.

The method for preparing anthranilic acid comprises the following steps: 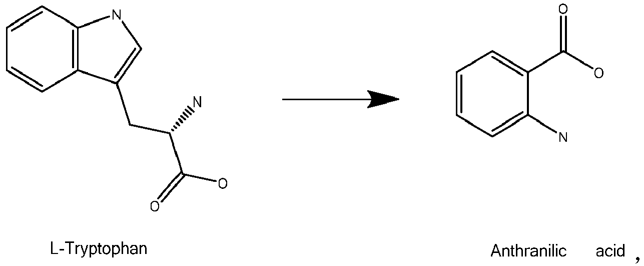 fermenting L-tryptophan by a microorganism or a fermentation product of the microorganisms to produce anthranilic acid.

The microorganism comprises at least one selected from the group consisting of *Corynebacterium sp., Bacillus sp., Lactobacillus sp., Lactococcus sp., Pseudomonas sp., Clostridium sp., Rhodococcus sp., Rhodobacter sp., Streptococcus sp., Saccharomyces sp., Aspergillus sp.* and *Streptomyces sp.*

In some embodiments, the microorganism is selected from *Saccharomyces sp.* Preferably, the microorganism is the *Saccharomyces cerevisiae* of the first aspect.

In some embodiments, the fermentation product of the microorganism is the fermentation product of the second aspect or the fermentation product obtained by the method of the third aspect.

In some embodiments, the method for preparing anthranilic acid comprises:
(1) preparing a seed culture: subjecting a strain of the microorganism to a at least one stage expanded culture or a culture by a solid slant culture medium and a seed culture medium sequentially to obtain the seed culture.
(2) inoculating the seed culture into a fermentation culture medium and culturing for 6 h to 12 h, then adding L-tryptophan to the culture medium in an amount of 10 g/L to 50 g/L and continuing to culture until the L-tryptophan is completely transformed into anthranilic acid, to obtain a biotransformation mixture;
(3) conducting post-processing procedure 1 on the biotransformation mixture to obtain anthranilic acid.

The process of continuing to culture until the L-tryptophan is completely transformed into anthranilic acid may be performed for 40 h to 60 h. In some embodiments, the process of continuing to culture until the L-tryptophan is completely transformed into anthranilic acid is performed for 45 h to 55 h.

In some embodiments, the method for preparing anthranilic acid comprises:
(1) preparing a seed culture: subjecting a strain of the microorganism to a at least one stage expanded culture or a culture by a solid slant culture medium and a seed culture medium sequentially to obtain the seed culture;
(2) inoculating the seed culture into a fermentation culture medium and culturing for 6 h to 12 h, then adding a first portion of L-tryptophan to induce the expression of enzymes related to an L-tryptophan degradation pathway, and then adding L-tryptophan in batches every 8-10 h and continuing to culture; after adding L-tryptophan for the last time, continuing to culture for 8 h-30 h to obtain a biotransformation mixture;
(3) conducting post-processing procedure 1 on the biotransformation mixture to obtain anthranilic acid.

The culture of the strain on the solid slant culture medium may be performed at a temperature of about 25 °C to about 30 °C. In some embodiments, the culture of the strain on the solid slant culture medium is performed at a temperature of about 28 °C to 30 °C.

The culture of the strain on the solid slant culture medium may be performed for about 40 h to about 60 h. In some embodiments, the culture of the strain on the solid slant culture medium is performed for 45 h to 55 h.

The culture of the strain in the seed culture medium may be performed at a temperature of about 28 °C to about 30°C. In some embodiments, the culture of the strain in the seed culture medium is performed at a temperature of 28 °C.

The culture of the strain in the seed culture medium may be a one-stage culture or a two-stage culture; the one-stage culture means that the strain is cultured once only in the seed culture medium; and the two-stage culture means that the strain is cultured once in the seed culture medium to obtain a one-stage seed culture, and then the one-stage seed culture is inoculated into a fresh seed culture medium to continue to culture once.

The one-stage culture may be performed for 4 h to 10 h. In some embodiments, the culture of the strain in the seed culture medium is performed for 4 h to 6 h.

Each culture of the two-stage culture may be performed for 8 h to 14 h. In some embodiments, the culture of the strain in the seed culture medium is performed for 10 h to 12 h.

The culture of the strain in the seed culture medium may be performed under stirring at a speed of 200 rpm to 250 rpm.

When the seed culture is inoculated into the fermentation culture medium, a volume ratio of the seed culture to the fermentation culture medium may be in a range of about 6:100 to about 12:100.

The fermentation culture medium may have a pH of about 5.0 to about 6.5.

The seed culture may have a pH of 5.0 to 6.5.

The culture in the fermentation culture medium in the step (2) may be performed at a temperature of 28°C to 30 °C.

The culture in the fermentation culture medium in the step (2) may be performed under stirring at a speed of 100 rpm to 600 rpm. In some embodiments, the culture in the fermentation culture medium in the step (2) may be performed under stirring at a speed of 200 rpm to 500 rpm. In some embodiments, the culture in the fermentation culture medium in the step (2) may be performed under stirring at a speed of 250 rpm to 400 rpm_{∘}

In the step (2), after the seed culture is inoculated into the fermentation culture medium, an aeration ratio in culture may be in a range of 1:0.2 to 1:1. In some embodiments, in the step (2), after the seed culture is inoculated into the fermentation culture medium, the aeration ratio in culture is in a range of 1:0.4 to 1:0.6. In some embodiments, in the step (2), after the seed culture is inoculated into the fermentation culture medium, the aeration ratio in culture is 1:0.5.

In some embodiments, the method for preparing anthranilic acid comprises:
(1) preparing a seed culture: coating a strain of the microorganism onto a solid slant culture medium and culturing at 28 °C for 48 h to obtain an activated strain; inoculating the activated strain into a seed culture medium and culturing to an early logarithmic phase at a temperature of 28 °C to 30 °C and under shaking at 200 rpm to 250 rpm to obtain the seed culture;
(2) inoculating the seed culture obtained in step (1) into a fermentation culture medium in a volume ratio of the seed culture to the fermentation culture medium of 6:100 to 12:100, culturing for 6-12 h at a pH of 5.0 to 6.5 and a temperature of 28 °C to 30 °C, under stirring at a speed of 100 rpm to 600 rpm and under an aeration ratio of 1:0.2 to 1:1, then adding a first portion of L-tryptophan to induce the expression of enzymes related to an L-tryptophan degradation pathway, and continuing to culture for 8-10 h and then adding a second portion of L-tryptophan and continuing to culture; adding L-tryptophan in batches and continuing to culture; and after adding L-tryptophan for the last time, continuing to culture for 8 h to 30 h to obtain a biotransformation mixture;
(3) conducting post-processing procedure 1 on the biotransformation mixture to obtain anthranilic acid.

In some embodiments, the aeration ratio is in a range of 1:0.4 to 1:0.6. In some embodiments, the aeration ratio is 1:0.5.

The first portion of L-tryptophan may be added in an amount of 1 g/L to 5 g/L. In some embodiments, the first portion of L-tryptophan is added in an amount of 2 g/L to 4 g/L, or 2 g/L to 3 g/L.

The second portion of L-tryptophan or each batch of L-tryptophan may be added in an amount of 5 g/L to 20 g/L. In some embodiments, the second portion of L-tryptophan or each batch of L-tryptophan is added in an amount of 10 g/L to 15 g/L.

The addition of L-tryptophan in batches may be performed as follows: after the second portion of L-tryptophan is added, the L-tryptophan is added once every 8-15 h in an amount of 5 g/L to 20 g/L. In some embodiments, the addition of L-tryptophan in batches may be performed as follows: after the second portion of L-tryptophan is added, the L-tryptophan is added once every 10-15 h in an amount of 5 g/L to 20 g/L. In some embodiments, the addition of L-tryptophan in batches may be performed as follows: after the second portion of L-tryptophan is added, the L-tryptophan is added once every 12 -15 h in an amount of 5 g/L to 20 g/L.

The total times of addition of L-tryptophan may be in a range of 2-8. In some embodiments, the total times of addition of L-tryptophan may be 2, 3, 4, 5, 6, 7 or 8.

In some embodiments, after L-tryptophan is added for the last time, culture is continued for 8 h to 30 h. In some embodiments, after L-tryptophan is added for the last time, culture is continued for 10 h to 30 h. In some embodiments, after L-tryptophan is added for the last time, culture is continued for 10 h to 25 h. In some embodiments, after L-tryptophan is added for the last time, culture is continued for 15 h to 25 h. In some embodiments, after L-tryptophan is added for the last time, culture is continued for 18 h to 25 h.

The post-processing procedure 1 may include: removing the strain by filtration, adjusting pH with sulfuric acid to precipitate anthranilic acid, filtering and collecting filter residues to obtain crude anthranilic acid, and recrystallizing the crude anthranilic acid using water as a solvent.

The adjustment of pH with sulfuric acid may include adjusting pH to be in a range of 2-3 with sulfuric acid.

Based on the total mass of the seed culture medium, the seed culture medium may comprise or consist of 1.5 wt% to 5 wt% of peptone, 0.5 wt% to 3 wt% of yeast extract, 2 wt% to 5 wt% of glucose, and solvent which is water. In some embodiments, based on the total mass of the seed culture medium, the seed culture medium comprises 2.0 wt% of peptone, 1.0 wt% of yeast extract, 2.0 wt% of glucose, and the rest of water.

Based on the total mass of the solid slant culture medium, the solid slant culture medium may comprise or consist of 1.5 wt% to 5 wt% of peptone, 0.5 wt% to 3 wt% of yeast extract, 2 wt% to 5 wt% of glucose, 1 wt% to 5 wt% of agar, and solvent which is water. In some embodiments, based on the total mass of the solid slant culture medium, the solid slant culture medium comprises 2.0 wt% of peptone, 1.0 wt% of yeast extract, 2.0 wt% of glucose, 2.0 wt% of agar, and the rest of water.

Based on the total mass of the fermentation culture medium, the fermentation culture medium may comprise or consist of 3.0 wt% to 5.0 wt% of glucose, 0.5 wt% to 1.0 wt% of ammonium dihydrogen phosphate, 0.2 wt% to 0.5 wt% of potassium dihydrogen phosphate, 0.1 wt% to 0.2 wt% of magnesium sulfate, 0.05 wt% to 0.1wt% of calcium sulfate, 0.2 wt% to 1.0 wt% of yeast extract powder, and solvent which is water. In some embodiments, based on the total mass of the fermentation culture medium, the fermentation culture medium comprises 4 wt% of glucose, 1 wt% of ammonium dihydrogen phosphate, 0.5 wt% of potassium dihydrogen phosphate, 0.2 wt% of magnesium sulfate, 0.1 wt% of calcium sulfate, 0.5 wt% of yeast extract powder, and the rest of water.

In the fifth aspect, a method for preparing methyl anthranilate is provided.

The method for preparing methyl anthranilate comprises esterifying the anthranilic acid obtained by the method of the second aspect to obtain methyl anthranilate.

The esterification may be conducted by any method in the prior art.

In some embodiments, the method for preparing methyl anthranilate comprises dissolving the anthranilic acid obtained by the method of the second aspect in methyl tert-butyl ether, adding natural methanol and Novozyme 435 thereto to obtain a mixture, subjecting the mixture to a reaction at 30-50 °C for 16 h to 20 h to obtain a product mixture, and conducting post-processing procedure 2 on the product mixture to obtain methyl anthranilate.

The post-processing procedure 2 may include: filtering and recovering Novozyme 435, and distilling the filtrate to recover methyl tert-butyl ether to obtain a residue; and rectifying the residue to obtain methyl anthranilate.

In the sixth aspect, use of the *Saccharomyces cerevisiae* of the first aspect or the fermentation product of the second aspect is provided.

Use of the *Saccharomyces cerevisiae* of the first aspect or the fermentation product of the second aspect in biodegrading L-tryptophan to prepare anthranilic acid is provided.

### Beneficial Effects

Compared with the prior art, one of the above technical solutions has at least one of the following beneficial technical effects:
(1) By using *Saccharomyces cerevisiae* OMK-77 in the above technical solutions to ferment and degrade L-tryptophan to prepare anthranilic acid, the space-time yield of anthranilic acid could reach 0.72 g/(L^{∗}h), and the product concentration could reach 30.2 g/L, which are much higher than those of the existing production processes; the production cost could be greatly reduced; and the subsequent production of methyl anthranilate could be realized through simple enzymatic esterification reaction. Thus, the above technical solutions have good industrial application prospects.
(2) By using *Saccharomyces cerevisiae* OMK-77 in the above technical solutions to ferment L-tryptophan to prepare anthranilic acid, the purity of the obtained anthranilic acid could be greater than 99%, and the yield of the anthranilic acid could be as high as 85%; the purity of the further prepared methyl anthranilate could be greater than 99%, and the yield of the methyl anthranilate could be as high as 78%.
(3) The anthranilic acid and the methyl anthranilate could be prepared by the methods of the above technical solutions, without the use of an organic solvent or with substantial reduction of the use of the organic solvent, and without chemical oxidants such as MnO₂. The methods have advantages such as fast reaction, highly safety, green and environmentally friendly process, fewer by-products, convenience in downstream extraction and purification, and fewer solvent residues.

### Definitions of terms:

Unless otherwise defined, all technical and scientific terms used in the present disclosure have the same meanings as those generally understood by those ordinary skilled in the art.

The term "aeration ratio" means the ratio of aeration volume per minute to the volume of fermentation broth. For example, the "aeration ratio of 1:0.4" means that the ratio of aeration volume per minute to the volume of fermentation broth is 1:0.4.

The term "weight percent" or "percent by weight" or "wt%" denotes a percentage obtained by dividing the weight of an individual component of a composition by the total weight of all components of the composition and then multiplying by 100.

The term "g/L" denotes a ratio of the weight of the described substance to the volume of the mixture containing the substance.

The term "rpm" means "revolutions per minute".

The term "room temperature" means indoor ambient temperature, which may be 15 °C to 30 °C, or 20 °C to 30 °C, or 25 °C to 30 °C, or about 25 °C.

In the present disclosure, all digits are approximate values, whether or not the wording "approximately" or "about" is used. Based on the digits disclosed, the value of each digit may have a difference within ±10% or considered to be reasonable by those skilled in the art, such as a difference within ±1%, ±2%, ±3%, ±4% or ±5%.

The term "natural methanol" means methanol obtained by fermentation, for example, methanol obtained from sucrose as raw material by fermentation.

The term "space-time yield" means the mass of anthranilic acid that can be obtained per unit volume of culture medium (including a seed culture medium and a fermentation culture medium) per unit time under given reaction conditions.

The terms "an embodiment", "some embodiments", "example", "specific example" or "some examples" intend to mean that specific features, structures, materials or characteristics illustrated in the embodiment or example are included in at least one embodiment or example of the present disclosure. In the present disclosure, exemplary statements for the above terms do not necessarily aim at the same embodiment or example. The described specific features, structures, materials or characteristics can be combined appropriately in any one or more embodiments or examples. In addition, without contradiction, those skilled in the art may combine or recombine different embodiments or examples and features of different embodiments or examples of the present disclosure.

### Description of Drawings

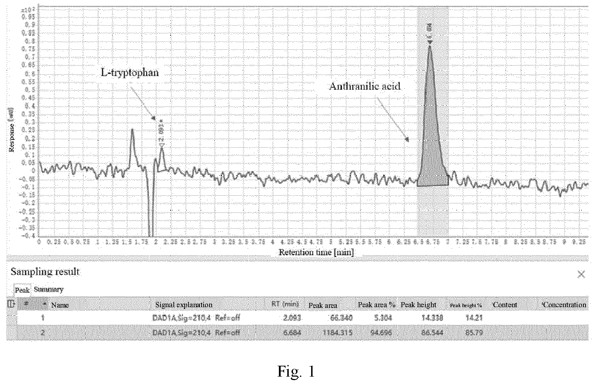

Fig. 1 shows a high pressure liquid chromatography (HPLC) result showing the progress of fermenting and degrading L-tryptophan by *Saccharomyces cerevisiae* OMK-77 to prepare natural anthranilic acid in Example 2.

### Detailed Description of Embodiments

In order to make those skilled in the art have a better understanding of the technical solutions in the present disclosure, some nonrestrictive examples are disclosed below for further describing the present disclosure in detail.

Reagents used in the present disclosure are commercially available or could be prepared by the methods described in the present disclosure.

### Example 1: Preparation of anthranilic acid (fermentation in a triangular shake flask)

### (1) Strain isolation

A strain that could degrade L-tryptophan to produce anthranilic acid was isolated from a surface of apple peel; and the strain was identified as *Saccharomyces cerevisiae* through 18S rDNA and ITS sequencing, and named as *Saccharomyces cerevisiae* OMK-77. The strain was collected at China Center for Type Culture Collection (Wuhan University, Wuhan, China) on October 18, 2021, with a collection Number of CCTCC M 20211292.

### (2) Strain activation

*Saccharomyces cerevisiae* OMK-77 was taken from -80 °C glycerol tube by streaking and coated onto a solid slant culture medium, and cultured at 28 °C for 48 h to obtain an activated strain.

The solid slant culture medium consisted of the following components based on mass percent: 2.0 wt% of peptone, 1.0 wt% of yeast extract, 2.0 wt% of glucose, 2.0wt% of agar, and the rest of water.

### (3) Preparation of a seed culture

The obtained activated strain was inoculated into a 500 mL triangular shake flask containing 30 mL of a seed culture medium, and cultured at 28 °C and 250 rpm for 4-6 h to obtain the seed culture.

The seed culture medium consisted of the following components based on mass percent: 2.0 wt% of peptone, 1.0 wt% of yeast extract, 2.0 wt% of glucose and the rest of water, and had an initial pH of 6.0, and was sterilized at 115 °C for 30 min.

### (4) Fermentation and biotransformation

5 mL of the seed culture was inoculated into a 500 mL triangular shake flask containing 50 mL of a fermentation culture medium, fermented and cultured at 28 °C and 250 rpm for 10 h. Then L-tryptophan was added thereto in an amount of 20 g/L, and fermentation was continued at 28 °C and 250 rpm for 48 h.

### (5) Precipitation and recrystallization.

After the fermentation, the operation temperature was increased to 60°C for inactivation of the strain and the fermentation product obtained in step (4) was centrifuged to remove the strain and obtain a fermentation supernatant. The fermentation supernatant was adjusted to have a pH of 2.0 with sulfuric acid to form a precipitate, and the precipitate was collected by centrifugation. The precipitate was then dissolved in an appropriate amount of hot water with a temperature of 90 °C, and then the resulting system was cooled to room temperature to precipitate anthranilic acid crystals. The crystals were dried to obtain 0.628 g of pure anthranilic acid with a purity of greater than 99% and a yield of 85%.

The fermentation culture medium consisted of the following components based on mass percent: 4 wt% of glucose, 1 wt% of ammonium dihydrogen phosphate, 0.5 wt% of potassium dihydrogen phosphate, 0.2 wt% of magnesium sulphate, 0.1 wt% of calcium sulphate, 0.5 wt% of yeast extract powder, and the rest of water, and had an initial pH of 6.0, and was sterilized at 115 °C for 30 min.

### Example 2: Preparation of methyl anthranilate (fermentation in a fermenter)

### Steps (1)-(2) were the same as those in Example 1.

(3) Preparation of a seed culture: the obtained activated strain was inoculated into a 500 mL triangular shake flask containing 50 mL of a seed culture medium, and cultured at 28 °C and 250 rpm for 10-12 h to obtain a primary seed culture. 50 mL of the primary seed culture was inoculated into a 10 L seed fermenter containing 6 L of the seed culture medium, and cultured at a temperature of 28°C, a stirring speed of 250 rpm and an aeration ratio of 1:0.5 for 10-12 h to obtain a secondary seed culture.

The seed culture medium had the same components as that in Example 1.

### (4) Fermentation and biotransformation

15.0 L of a fermentation culture medium was introduced to a 30 L fermenter, and sterilized at 115 °C for 30 min. The secondary seed culture was inoculated into the 30 L fermenter at a 10% inoculation ratio and cultured at temperature of 28 °C, a stirring speed of 400 rpm and an aeration ratio of 1:0.5. After culturing (i.e., fermenting) for 8 h, L-tryptophan was added thereto in an amount of 2 g/L, and fermentation was continued. Subsequently, L-tryptophan was further added in an amount of 15 g/L respectively at 16 h, 28 h and 40 h of the fermentation (the fermentation time was defined as the time elapsed after the secondary seed culture was inoculated into the 30 L fermenter). The total fermentation time was 58 h. (Fig. 1 shows a reaction progress of fermentation for 58 h. As can be seen, L-tryptophan is almost completely converted into anthranilic acid, with a space-time yield of 0.72 g/(L^{∗}h), and a product concentration of 30.2 g/L).

### (5) Precipitation and recrystallization

After the fermentation, the operation temperature was increased to 60 °C for inactivation of the strain and the fermentation product obtained in step (4) was filtered to remove the strain and obtain a fermentation supernatant. The fermentation supernatant was adjusted to have a pH of 2.0 with sulfuric acid to form a precipitate, and the precipitate was collected by filtration. The precipitate was then dissolved in an appropriate amount of hot water at 90 °C, and then the resulting system was cooled to room temperature to precipitate anthranilic acid crystals. The crystals were dried to obtain 458.3 g of pure anthranilic acid with a purity of greater than 99% and a yield of 88%.

### (6) Esterification

458.3 g of the anthranilic acid crystals obtained in step (5) were dissolved in 7 L of methyl tert-butyl ether; and 350 g of Novozyme 435 and 1 kg of activated zeolite 3A were added thereto. The resulting system was stirred, and reacted at a temperature of 45 °C. During the reaction, 172 mL of natural methanol was added at the time of 0 h, 4 h, 9 h and 15 h respectively. The reaction was performed for a total time of 22 h, to obtain a biotransformation mixture.

### (7) Recovery of catalyst and solvent and rectification

The biotransformation mixture obtained in step (6) was filtered to recover Novozyme 435 and zeolite 3A; and the obtained clear solution was transferred into a distillation flask for recovery of methyl tert-butyl ether. The obtained residue was transferred into a rectifying tower for rectification. The rectification temperature was controlled, and fractions at appropriate temperature were collected, to obtain 447.8 g of methyl anthranilate with a purity of greater than 99% and a total yield of 78%.

The fermentation culture medium had the same components as that in Example 1.

The technical solutions of the present disclosure are described through the preferred examples. Those skilled in the art may obviously modify or appropriately change and combine the methods and use described herein without departing from the scope and concept of the present disclosure to realize and apply the technical solutions of the present disclosure. Those skilled in the art may appropriately adjust the process parameters to realize the technical solutions of the present disclosure in light of the contents disclosed herein. In particular, all similar substitutions and modifications are obvious to those skilled in the art and shall be deemed to fall within the scope of the present disclosure.

## Claims

1. A *Saccharomyces cerevisiae,* having a collection number of CCTCC M 20211292.

2. A fermentation product of the *Saccharomyces cerevisiae* of claim 1.

3. A method for preparing the fermentation product of claim 2, comprising: activating the *Saccharomyces cerevisiae* of claim 1, subjecting the *Saccharomyces cerevisiae* to a at least one stage expanded culture, and collecting fermentation product.

4. A method for preparing anthranilic acid, comprising the following steps: fermenting L-tryptophan by a microorganism or a fermentation product of the microorganism to produce the anthranilic acid.

5. The method according to claim 4, wherein the microorganism comprises at least one selected from the group consisting of *Corynebacterium sp., Bacillus sp., Lactobacillus sp., Lactococcus sp., Pseudomonas sp., Clostridium sp., Rhodococcus sp., Rhodobacter sp., Streptococcus sp., Saccharomyces sp., Aspergillus sp.* and *Streptomyces sp.;* preferably, the microorganism is the *Saccharomyces cerevisiae* of claim 1.

6. The method according to any one of claims 4-5, comprising:
(1) preparing a seed culture: subjecting a strain of the microorganism to a at least one stage expanded culture or a culture by a solid slant culture medium and a seed culture medium sequentially to obtain the seed culture;
(2) inoculating the seed culture into a fermentation culture medium and culturing for 6 h to12 h, then adding L-tryptophan to the culture medium in an amount of 10 g/L to 50 g/L and continuing to culture until the L-tryptophan is completely transformed into anthranilic acid, to obtain a biotransformation mixture; or
inoculating the seed culture into a fermentation culture medium and culturing for 6 h to12 h, then adding a first portion of L-tryptophan to induce the expression of enzymes related to an L-tryptophan degradation pathway, and then adding L-tryptophan in batches every 8-10 h and continuing to culture; after adding L-tryptophan for the last time, continuing to culture for 8 h to 30 h to obtain a biotransformation mixture;
(3) conducting post-processing procedure 1 on the biotransformation mixture to obtain anthranilic acid.

7. The method according to any one of claims 4-6, wherein based on the total mass of the seed culture medium, the seed culture medium comprises 1.5 wt% to 5 wt% of peptone, 0.5 wt% to 3 wt% of yeast extract, 2 wt% to 5 wt% of glucose, and a solvent which is water; and/or
based on the total mass of the solid slant culture medium, the solid slant culture medium comprises 1.5 wt% to 5 wt% of peptone, 0.5 wt% to 3 wt% of yeast extract, 2 wt% to 5 wt% of glucose, 1 wt% to 5 wt% of agar, and a solvent which is water; and/or
based on the total mass of the fermentation culture medium, the fermentation culture medium comprises 3.0 wt% to 5.0 wt% of glucose, 0.5 wt% to 1.0 wt% of ammonium dihydrogen phosphate, 0.2 wt% to 0.5 wt% of potassium dihydrogen phosphate, 0.1 wt% to 0.2 wt% of magnesium sulfate, 0.05 wt% to 0.1 wt% of calcium sulfate, 0.2 wt% to 1.0 wt% of yeast extract powder, and a solvent which is water.

8. A method for preparing methyl anthranilate, comprising esterifying the anthranilic acid obtained by the method of any one of claims 4-7 to obtain methyl anthranilate.

9. The method according to claim 8, comprising dissolving the anthranilic acid obtained by the method of claim 6 into methyl tert-butyl ether, then adding natural methanol and Novozyme 435 to obtain a mixture, subjecting the mixture to a reaction at 30-50 °C for 16 h to 20 h to obtain a product mixture, and conducting post-processing procedure 2 on the product mixture to obtain methyl anthranilate.

10. Use of the *Saccharomyces cerevisiae* of claim 1 or the fermentation product of the *Saccharomyces cerevisiae* of claim 2 in biodegrading L-tryptophan to prepare anthranilic acid.
